# EUROPEAN PATENT APPLICATION

(11) **EP 4 517 878 A1**
(43) Date of publication of application: **05.03.2025**
(21) Application number: 22952467.3
(22) Date of filing: 29.07.2022
(51) Int. Cl.: H01M 4/40, H01M 10/056

(54) **SODIUM-ION BATTERY ELECTROLYTE, SECONDARY BATTERY, BATTERY MODULE, BATTERY PACK, AND ELECTRIC DEVICE**

(71) Applicant: Contemporary Amperex Technology (Hong Kong) Limited, Hong Kong (HK)
(72) Inventor: QIN, Meng, Ningde City, Fujian 352100 (CN); GUAN, Yingjie, Ningde City, Fujian 352100 (CN); MA, Qingyan, Ningde City, Fujian 352100 (CN); ZHAO, Yuzhen, Ningde City, Fujian 352100 (CN); WEN, Yan, Ningde City, Fujian 352100 (CN); HUANG, Qisen, Ningde City, Fujian 352100 (CN)
(74) Representative: Gong, Jinping
(86) International application number: PCT/CN2022/108955
(87) International publication number: WO 2024/021019

(57) **Abstract**

The present application provides an electrolyte solution for a sodium-ion battery, comprising a sodium salt of formula NaBOₐFₓR_{y}^{-z}, a fluoroalkyl ether, and other ethers except for the fluoroalkyl ether, wherein a, x, y, and z are as defined in the specification. The electrolyte solution according to the present application has good stability, particularly oxidation resistance and good solvability of sodium ions, thus broadening an electrochemical window of a corresponding sodium-ion battery, enhancing coulombic efficiency, and improving cycling performance and safety performance. The present application further provides a secondary battery, a battery module, a battery pack, and an electrical apparatus using the electrolyte solution for a sodium-ion battery.

## Description

### Technical Field

The present application relates to the technical field of secondary batteries, and particularly relates to an electrolyte solution for a sodium-ion battery, and a secondary battery, a battery module, a battery pack, and an electrical apparatus using the same.

### Background

In recent years, the demand for lithium-ion batteries is increasing, but limited lithium resources restrict sustainable development of the lithium-ion batteries. As an important supplement to the lithium-ion batteries, sodium-ion secondary batteries have attracted more and more attention. As a transmission carrier of sodium ions in the battery, an electrolyte solution for a sodium-ion battery can improve or enhance various performances of the battery, and is an important component of the sodium-ion battery. However, the electrolyte solution for a sodium-ion battery used in the prior art has poor stability, particularly poor oxidation resistance, and poor solvability of sodium ions, particularly at a high potential, thereby failing to contribute to broadening an electrochemical window of the sodium-ion battery, failing to enhance coulombic efficiency, and failing to improve cycling performance and safety performance. Therefore, how to provide an electrolyte solution that not only has good stability, but also can effectively regulate the solvability of sodium ions is still a difficult problem to be urgently solved by those skilled in the art.

### Summary of the Invention

In view of the above problems, an object of the present application is to provide an electrolyte solution that has good stability, particularly oxidation resistance and good solvability of sodium ions, thus broadening an electrochemical window of a corresponding sodium-ion battery, enhancing coulombic efficiency, and improving cycling performance and safety performance.

For the above purpose, a first aspect of the present application provides an electrolyte solution for a sodium-ion battery, comprising:
a sodium salt of formula NaBOₐFₓR_{y}^{-z}, wherein 2a+x+y×z=2 or 4, 0≤a≤3, 0≤x≤4, 0≤y, 0≤z, 0≤y×z≤4, and R is selected from one or more of oxalato 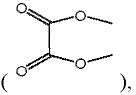 phenyl, cyano, C₁-C₆-fluoroalkyl such as trifluoromethyl, pentafluoroethyl, or 2,2,2-trifluoroethyl, or C₁-C₆-alkoxy such as methoxy or ethoxy;
a fluoroalkyl ether; and
other ethers except for the fluoroalkyl ether, wherein optionally, the other ethers are selected from one or more of an aliphatic ether with 4-20 carbon atoms, an alicyclic ether with 3-8 carbon atoms, an aromatic ether with 7-20 carbon atoms, or a crown ether.

The electrolyte solution according to the present application has good stability and solvability of sodium ions, thereby contributing to broadening an electrochemical window of a corresponding secondary battery, enhancing coulombic efficiency, and improving cycling performance and safety performance.

In any of embodiments, optionally, the electrolyte solution for a sodium-ion battery satisfies one or more conditions of:
(1) a content of the sodium salt of formula NaBOₐFₓR_{y}^{-z} is 5-40 wt%, optionally 10-20 wt%;
(2) a content of the fluoroalkyl ether is 1-82 wt%, optionally 1-48 wt%, more optionally 1-20 wt%, and most optionally 2-8 wt%;
(3) a sum of the content of the sodium salt of formula NaBOₐFₓR_{y}^{-z} and the content of the fluoroalkyl ether is 10-95 wt%, optionally 10-60 wt%, and more optionally 12-28 wt%;
(4) a content of the other ethers is 5-90 wt%, optionally 40-90 wt%, and more optionally 72-88 wt%; and
(5) a concentration of the sodium salt of formula NaBOₐFₓR_{y}^{-z} is 0.5-8 mol/L, optionally 1-4 mol/L;
wherein the contents in (1)-(4) are all based on a total weight of the electrolyte solution for a sodium-ion battery, and a total weight of the components in the electrolyte solution for a sodium-ion battery is 100 wt%.

When the electrolyte solution for a sodium-ion battery satisfies one or more of the above conditions, it is beneficial to further broadening the electrochemical window of the corresponding secondary battery, enhancing the coulombic efficiency, and improving the cycling performance and safety performance.

In any of embodiments, optionally, a mass ratio of the sodium salt of formula NaBOₐFₓR_{y}^{-z} to the fluoroalkyl ether is 1:0.08-6, optionally 1:0.08-4, and more optionally 1:0.1-0.8; and
optionally, a mass ratio of the fluoroalkyl ether to the other ethers except for the fluoroalkyl ether is 1:0.06-90, optionally 1:2-90, and more optionally 1:9-44.

When a ratio of the components in the electrolyte solution for a sodium-ion battery satisfies one or more of the above conditions, it is beneficial to further broadening the electrochemical window of the corresponding secondary battery, enhancing the coulombic efficiency, and improving the cycling performance and safety performance.

In any of embodiments, optionally, the sodium salt of formula NaBOₐFₓR_{y}^{-z} is selected from one or more of sodium difluoroborate, sodium tetrafluoroborate, sodium bis(oxalato)borate, sodium difluoro(oxalato)borate, sodium tetraphenylborate, sodium tetracyanoborate, sodium tetrakis(trifluoromethyl)borate, sodium bis(trifluoromethyl)difluoroborate, sodium pentafluroroethyltrifluoroborate, sodium dicyano(oxalato)borate, sodium methoxytricyanoborate, sodium ethoxytricyanoborate, sodium tetramethooxyborate, sodium tetraethoxyborate, or sodium cyanotris(2,2,2-trifluoroethyl)borate; and
is optionally one or more of sodium bis(oxalato)borate, sodium difluoro(oxalato)borate, sodium tetrakis(trifluoromethyl)borate, sodium bis(trifluoromethyl)difluoroborate, or sodium dicyano(oxalato)borate.

An appropriate sodium salt of formula NaBOₐFₓR_{y}^{-z} is selected, thereby contributing to further broadening the electrochemical window of the corresponding secondary battery, enhancing the coulombic efficiency, and improving the cycling performance and safety performance.

In any of embodiments, optionally, the fluoroalkyl ether is selected from one or more of 1,1,2,2-tetrafluoroethyl-2,2,3,3-tetrafluoropropyl ether, bis(2,2,2-trifluoroethyl) ether, 1,1,2,2-tetrafluoroethyl-2,2,2-trifluoroethyl ether, methoxynonafluorobutane, or ethoxynonafluorobutane.

In any of embodiments, optionally, the other ethers are selected from one or more of ethylene glycol diethyl ether, ethylene glycol dimethyl ether, diethylene glycol dimethyl ether, tridiethylene glycol dimethyl ether, tetraethylene glycol dimethyl ether, 1,3-dioxolane, tetrahydrofuran, methyltetrahydrofuran, diphenyl ether, or crown ether; and
are optionally one or more of ethylene glycol diethyl ether, ethylene glycol dimethyl ether, or diethylene glycol dimethyl ether.

An appropriate fluoroalkyl ether and other appropriate ethers are selected, thereby contributing to further broadening the electrochemical window of the corresponding secondary battery, enhancing the coulombic efficiency, and improving the cycling performance and safety performance.

In any of embodiments, optionally, the electrolyte solution for a sodium-ion battery comprises no carbonate; or if a carbonate is present, a ratio of a percentage mass content of the carbonate to a percentage mass content of all ether solvents is from 1:9 to 3:2, optionally from 1:6 to 1:1.

A second aspect of the present application provides a sodium-ion secondary battery, comprising the electrolyte solution for a sodium-ion battery in the first aspect of the present application.

A third aspect of the present application provides a battery module, comprising the sodium-ion secondary battery in the second aspect of the present application.

A fourth aspect of the present application provides a battery pack, comprising at least one of the sodium-ion secondary battery in the second aspect of the present application or the battery module in the third aspect of the present application.

A fifth aspect of the present application provides an electrical apparatus, comprising at least one of the sodium-ion secondary battery in the second aspect of the present application, the battery module in the third aspect of the present application, or the battery pack in the fourth aspect of the present application.

### [Beneficial effects]

The electrolyte solution for a sodium-ion battery according to the present application comprises a sodium salt of formula NaBOₐFₓR_{y}^{-z}, a fluoroalkyl ether, and other ethers except for the fluoroalkyl ether. The synergistic effects of the above materials can improve the solvability of sodium ions, improve the stability, particularly the oxidation resistance, of the electrolyte solution, and improve the self-discharge of the secondary battery in a high temperature environment, thereby improving the cycling performance of the corresponding secondary battery, broadening the electrochemical window, and enhancing the coulombic efficiency.

The battery module, the battery pack, and the electrical apparatus according to the present application comprise the sodium-ion secondary battery according to the present application, and thus have at least same advantages as the secondary battery.

### Description of Drawings

Fig. 1 is a schematic diagram of a secondary battery according to an embodiment of the present application.
Fig. 2 is an exploded view of the secondary battery according to an embodiment of the present application shown in Fig. 1.
Fig. 3 is a schematic diagram of a battery module according to an embodiment of the present application.
Fig. 4 is a schematic view of a battery pack according to an embodiment of the present application.
Fig. 5 is an exploded view of the battery pack according to an embodiment of the present application shown in Fig. 4.
Fig. 6 is a schematic diagram of an electrical apparatus in which a secondary battery is used as a power source according to an embodiment of the present application.

Description of reference numerals:
1 battery pack; 2 upper box; 3 lower box; 4 battery module; 5 secondary battery; 51 case; 52 electrode assembly; 53 top cover assembly

### Detailed Description

Hereinafter, embodiments of an electrolyte solution for a sodium-ion battery, a secondary battery using the same, a battery module, a battery pack, and an electrical apparatus of the present application are specifically disclosed by referring to the detailed description of drawings as appropriate. However, there may be cases where unnecessary detailed description is omitted. For example, there are cases where detailed descriptions of well-known items and repeated descriptions of actually identical structures are omitted. This is to avoid unnecessary redundancy in the following descriptions and to facilitate understanding by those skilled in the art. In addition, the drawings and subsequent descriptions are provided for those skilled in the art to fully understand the present application, and are not intended to limit the subject matter recited in the claims.

"Ranges" disclosed in the present application are defined in the form of lower limits and upper limits, a given range is defined by the selection of a lower limit and an upper limit, and the selected lower limits and upper limits define boundaries of a particular range. A range defined in this manner may be inclusive or exclusive of end values, and may be randomly combined, that is, any lower limit may be combined with any upper limit to form a range. For example, if the ranges of 60-120 and 80-110 are listed for a particular parameter, it is understood that the ranges of 60-110 and 80-120 are also contemplated. Additionally, if the minimum range values 1 and 2 are listed, and if the maximum range values 3, 4, and 5 are listed, the following ranges are all contemplated: 1-3, 1-4, 1-5, 2- 3, 2-4, and 2-5. In the present application, unless stated otherwise, the numerical range "a-b" represents an abbreviated representation of any combination of real numbers between a to b, where both a and b are real numbers. For example, the numerical range "0-5" means that all real numbers between "0-5" have been listed herein, and "0-5" is just an abbreviated representation of the combination of these numerical values. In addition, when a parameter is expressed as an integer greater than or equal to 2, it is equivalent to disclosing that the parameter is, for example, an integer of 2, 3, 4, 5, 6, 7, 8, 9, 10, 11, 12, and the like.

Unless otherwise specified, all embodiments and optional embodiments of the present application may be combined with each other to form new technical solutions.

Unless otherwise specified, all technical features and optional technical features of the present application may be combined with each other to form new technical solutions.

Unless otherwise specified, all steps of the present application may be performed sequentially or randomly, and preferably sequentially. For example, the method includes steps (a) and (b), which means that the method may include steps (a) and (b) performed sequentially, or may include steps (b) and (a) performed sequentially. For example, the reference to the method may further comprise step (c), which means that step (c) may be added to the method in any order, for example, the method may comprise steps (a), (b) and (c), or may comprise steps (a), (c) and (b), or may comprise steps (c), (a) and (b), and so on.

Unless otherwise specifically stated, the terms "include/including" and "comprise/comprising" mentioned in the present application may be open-ended, or may be closed-ended. For example, the "including" and "comprising" may indicate that it is also possible to include or comprise other components not listed, and it is also possible to include or comprise only the listed components.

Unless otherwise specifically stated, the term "or" is inclusive in the present application. By way of example, the phrase "A or B" means "A, B, or both A and B." More specifically, the condition "A or B" is satisfied under any one of the following conditions: A is true (or present) and B is false (or absent); A is false (or absent) and B is true (or present); or both A and B are true (or present).

The inventors found during studies that the existing electrolyte solution for a sodium-ion battery is not satisfactory in terms of stability, particularly oxidation resistance, which is not conducive to improving the performance of the corresponding secondary battery. The inventors unexpectedly found through a lot of studies that a sodium salt of formula NaBOₐFₓR_{y}^{-z} and an ether compound (including a fluoroalkyl ether and other ethers) are both comprised in an electrolyte solution, thereby contributing to synergistic effects of various materials, significantly broadening an electrochemical window of a corresponding secondary battery, enhancing coulombic efficiency, and improving cycling performance and safety performance. In addition, the inventors found through further studies on a type, a content, and a material ratio of each material that the battery performance can be further improved by regulating these factors.

### [Electrolyte solution]

The first aspect of the present application provides an electrolyte solution for a sodium-ion battery, comprising:
a sodium salt of formula NaBOₐFₓR_{y}^{-z}, wherein 2a+x+y×z=2 or 4, 0≤a≤3, 0≤x≤4, 0≤y, 0≤z, 0≤y×z≤4, and R is selected from one or more of oxalato 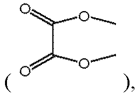 phenyl, cyano, C₁-C₆-fluoroalkyl such as trifluoromethyl, pentafluoroethyl, or 2,2,2-trifluoroethyl, or C₁-C₆-alkoxy such as methoxy or ethoxy;
a fluoroalkyl ether; and
other ethers except for the fluoroalkyl ether, wherein optionally, the other ethers are selected from one or more of an aliphatic ether with 4-20 carbon atoms, an alicyclic ether with 3-8 carbon atoms, an aromatic ether with 7-20 carbon atoms, or a crown ether.

The electrolyte solution for a sodium-ion battery according to the present application comprises a sodium salt of formula NaBOₐFₓR_{y}^{-z}, a fluoroalkyl ether, and other ethers except for the fluoroalkyl ether. In the electrolyte solution, the ether solvent can form a solvated structure with the sodium salt of formula NaBOₐFₓR_{y}^{-z} to improve solvability of sodium ions, thereby contributing to promoting free migration of the sodium ions, reducing consumption of active sodium ions, and improving the cycling performance of the corresponding battery.

The electrolyte solution according to the present application comprises the sodium salt of formula NaBOₐFₓR_{y}^{-z}, which helps to reduce self-discharge of the corresponding secondary battery in a high-temperature environment, and improve the capacity retention rate, thereby enhancing the coulombic efficiency.

The electrolyte solution according to the present application comprises a fluoroalkyl ether, which has strong oxidation resistance, can associate with boronic anions, and can reduce the number of other ether molecules in the formed solvated structure, thereby greatly improving the stability, particularly the oxidation resistance, of the electrolyte solution. The improvement of the stability, particularly the oxidation resistance, of the electrolyte solution helps to broaden the electrochemical window of the corresponding secondary battery.

Further, the fluoroalkyl ether introduces a large number of fluorine ions into the electrolyte solution, thereby promoting the formation of an NaF-rich stable interface film on a surface of a negative electrode. The NaF-rich stable interface film is beneficial to inhibiting side reactions, thereby preventing side reaction products from covering on the surface of the negative electrode, preventing blocking of sodium ion migration channels from resulting in sodium ion aggregation, and then reducing the production of sodium dendrite, which is not only beneficial to improving the safety performance of the corresponding secondary battery, but also beneficial to reducing the consumption of active sodium ions, thus improving the cycling performance.

In addition, ether solvent (including the fluoroalkyl ether) molecules can build a stable electrode/electrolyte solution interface on surfaces of a sodium metal negative electrode (including negative electrode free), a carbon material negative electrode, and other non-carbon material negative electrodes, thus forming a stable solid electrolyte interface (SEI), and reducing electrochemical polarization. Particularly, for a graphite system, the sodium ions and the ether solvent molecules can form a stable ternary graphite intercalation compound through a highly reversible co-intercalation reaction in graphite, thereby enhancing the negative electrode stability of the corresponding secondary battery.

It should be noted that, in the sodium salt of formula NaBOₐFₓR_{y}^{-z}, "a" satisfies 0≤a≤3, and is optionally 0. -z is a valence state of group R, for example, -z is -1 when R is cyano; -z is -1 when R is methoxy or ethoxy; and -z is -2 when R is oxalato.

In a more optional embodiment, a, x, y, and z are all integers.

It should be noted that those skilled in the art understand that in the electrolyte solution according to the present application, the fluoroalkyl ether and other ethers are both present, but there are no specific requirements for a ratio of the two. The two mixed at any ratio can achieve the technical purpose of the present application, but the fluoroalkyl ether is more expensive. In order to further optimize the technical effects and consider the cost benefits, the inventors studied the content and ratio of each component, as described below.

In some embodiments, optionally, the electrolyte solution for a sodium-ion battery according to the present application satisfies one or more conditions of:
(1) a content of the sodium salt of formula NaBOₐFₓR_{y}^{-z} is 5-40 wt%, optionally 10-20 wt%;
(2) a content of the fluoroalkyl ether is 1-82 wt%, optionally 1-48 wt%, more optionally 1-20 wt%, and most optionally 2-8 wt%;
(3) a sum of the content of the sodium salt of formula NaBOₐFₓR_{y}^{-z} and the content of the fluoroalkyl ether is 10-95 wt%, optionally 10-60 wt%, and more optionally 12-28 wt%;
(4) a content of the other ethers is 5-90 wt%, optionally 40-90 wt%, and more optionally 72-88 wt%; and
(5) a concentration of the sodium salt of formula NaBOₐFₓR_{y}^{-z} is 0.5-8 mol/L, optionally 1-4 mol/L;
wherein the contents in (1)-(4) are all based on a total weight of the electrolyte solution for a sodium-ion battery, and a total weight of the components in the electrolyte solution for a sodium-ion battery is 100 wt%.

When the electrolyte solution for a sodium-ion battery satisfies one or more of the above conditions, it is beneficial to further broadening the electrochemical window of the corresponding secondary battery, enhancing the coulombic efficiency, and improving the cycling performance and safety performance.

As an example, based on the total weight of the electrolyte solution for a sodium-ion battery, the content of the sodium salt of formula NaBOₐFₓR_{y}^{-z} may be 5 wt%, 10 wt%, 11.5 wt%, 12 wt%, 12.5 wt%, 14.5 wt%, 20 wt%, 23 wt%, or 40 wt%, or a range consisting of any two thereof.

As an example, based on the total weight of the electrolyte solution for a sodium-ion battery, the content of the fluoroalkyl ether may be 1 wt%, 2 wt%, 5 wt%, 8 wt%, 20 wt%, 48 wt%, or 82 wt%, or a range consisting of any two thereof.

As an example, based on the total weight of the electrolyte solution for a sodium-ion battery, the sum of the content of the sodium salt of formula NaBOₐFₓR_{y}^{-z} and the content of the fluoroalkyl ether may be 10 wt%, 12 wt%, 12.7 wt%, 13.6 wt%, 15 wt%, 16.5 wt%, 17.5 wt%, 20 wt%, 25 wt%, 28 wt%, 32 wt%, 48 wt%, 60 wt%, or 95 wt%, or a range consisting of any two thereof.

As an example, based on the total weight of the electrolyte solution for a sodium-ion battery, the content of the other ethers may be 5 wt%, 40 wt%, 68 wt%, 72 wt%, 75 wt%, 82.5 wt%, 83.5 wt%, 85 wt%, 86.4 wt%, 87.3 wt%, 88 wt%, or 90 wt%, or a range consisting of any two thereof.

As an example, the concentration of the sodium salt of formula NaBOₐFₓR_{y}^{-z} may be 0.5 M (i.e., mol/L), 1 M, 1.5 M, 4 M, or 8 M, or a range consisting of any two thereof.

In some embodiments, optionally, a mass ratio of the sodium salt of formula NaBOₐFₓR_{y}^{-z} to the fluoroalkyl ether is 1:0.08-6, optionally 1:0.08-4, and more optionally 1:0.1-0.8. As an example, the mass ratio of the sodium salt of formula NaBOₐFₓR_{y}^{-z} to the fluoroalkyl ether may be 1:0.08, 1:0.1, 1:0.125, 1:0.17, 1:0.2, 1:0.25, 1:0.4, 1:0.44, 1:0.5, 1:0.66, 1:0.8, 1:1, 1:3.8, 1:4, 1:5.6, or 1:6, or a range consisting of any two thereof.

In some embodiments, optionally, a mass ratio of the fluoroalkyl ether to the other ethers except for the fluoroalkyl ether is 1:0.06-90, optionally 1:2-90, and more optionally 1:9-44. As an example, the mass ratio of the fluoroalkyl ether to the other ethers except for the fluoroalkyl ether may be 1:0.06, 1:0.84, 1:2, 1:3.4, 1:9, 1:10, 1:11, 1:14.4, 1:15, 1:16.5, 1:16.7, 1:18, 1:43.2, 1:44, 1:87.3, or 1:90, or a range consisting of any two thereof.

When a ratio of the components in the electrolyte solution for a sodium-ion battery satisfies one or more of the above conditions, it is beneficial to further broadening the electrochemical window of the corresponding secondary battery, enhancing the coulombic efficiency, and improving the cycling performance and safety performance.

In some embodiments, optionally, the sodium salt of formula NaBOₐFₓR_{y}^{-z} is selected from one or more of sodium difluoroborate, sodium tetrafluoroborate, sodium bis(oxalato)borate, sodium difluoro(oxalato)borate, sodium tetraphenylborate, sodium tetracyanoborate, sodium tetrakis(trifluoromethyl)borate, sodium bis(trifluoromethyl)difluoroborate, sodium pentafluroroethyltrifluoroborate, sodium dicyano(oxalato)borate, sodium methoxytricyanoborate, sodium ethoxytricyanoborate, sodium tetramethooxyborate, sodium tetraethoxyborate, or sodium cyanotris(2,2,2-trifluoroethyl)borate; and
is optionally one or more of sodium bis(oxalato)borate, sodium difluoro(oxalato)borate, sodium tetrakis(trifluoromethyl)borate, sodium bis(trifluoromethyl)difluoroborate, or sodium dicyano(oxalato)borate.

An appropriate sodium salt of formula NaBOₐFₓR_{y}^{-z} is selected, thereby contributing to further broadening the electrochemical window of the corresponding secondary battery, enhancing the coulombic efficiency, and improving the cycling performance and safety performance.

In some embodiments, optionally, the fluoroalkyl ether is selected from one or more of 1,1,2,2-tetrafluoroethyl-2,2,3,3-tetrafluoropropyl ether, bis(2,2,2-trifluoroethyl) ether, 1,1,2,2-tetrafluoroethyl-2,2,2-trifluoroethyl ether, methoxynonafluorobutane, or ethoxynonafluorobutane.

In some embodiments, optionally, the other ethers are selected from one or more of ethylene glycol diethyl ether, ethylene glycol dimethyl ether, diethylene glycol dimethyl ether, tridiethylene glycol dimethyl ether, tetraethylene glycol dimethyl ether, 1,3-dioxolane, tetrahydrofuran, methyltetrahydrofuran, diphenyl ether, or crown ether; and is optionally one or more of ethylene glycol diethyl ether, ethylene glycol dimethyl ether, or diethylene glycol dimethyl ether.

An appropriate fluoroalkyl ether and other appropriate ethers are selected, thereby contributing to further broadening the electrochemical window of the corresponding secondary battery, enhancing the coulombic efficiency, and improving the cycling performance and safety performance.

In some embodiments, optionally, the fluoroalkyl ether and the other ethers are liquid at room temperature.

In some embodiments, optionally, the electrolyte solution for a sodium-ion battery comprises no carbonate; or if a carbonate is present, a ratio of a percentage mass content of the carbonate to a percentage mass content of all ether solvents is from 1:9 to 3:2, optionally from 1:6 to 1:1.

When the electrolyte solution comprises no carbonate; or a mass ratio of the carbonate to the ether solvents is within the above range, it is beneficial to further broadening the electrochemical window of the corresponding secondary battery, enhancing the coulombic efficiency, and improving the cycling performance and safety performance.

It should be noted that the electrolyte solution for a sodium-ion battery according to the present application can be prepared using a method commonly used in the art. As an example, the electrolyte solution according to the present application can be prepared by: adding the sodium salt of formula NaBOₐFₓR_{y}^{-z} and the fluoroalkyl ether into other ethers except for the fluoroalkyl ether in an inert gas atmosphere, and sufficiently mixing the mixture by stirring, so that the concentration of the sodium salt of formula NaBOₐFₓR_{y}^{-z} is 0.5-8 mol/L, optionally 1-4 mol/L.

### [Secondary battery]

The second aspect of the present application provides a sodium-ion secondary battery, comprising the electrolyte solution in the first aspect of the present application. In general, the secondary battery not only comprises the electrolyte solution, but also comprises a positive electrode plate, a negative electrode plate, and a separator.

The secondary battery may be prepared using a method commonly used in the art, for example, by making the positive electrode plate, the negative electrode plate, and the separator into an electrode assembly using a winding process or a stacking process, then injecting the electrolyte solution into the electrode assembly, and sealing the electrode assembly, to provide the secondary battery.

The above components of the secondary battery are described below respectively.

### [Positive electrode plate]

The positive electrode plate comprises a positive electrode current collector and a positive electrode film layer arranged on at least one surface of the positive electrode current collector, and the positive electrode film layer comprises a positive electrode active material.

As an example, the positive electrode current collector has two surfaces opposite in its own thickness direction, and the positive electrode film layer is arranged on either one or both of the two opposite surfaces of the positive electrode current collector.

In some embodiments, the positive electrode current collector can be a metal foil or a composite current collector. For example, an aluminum foil can be used as the metal foil. The composite current collector may comprise a high molecular material substrate and a metal layer formed on at least one surface of the polymer material substrate. The composite current collector may be formed by forming a metal material (such as aluminum, aluminum alloy, nickel, nickel alloy, titanium, titanium alloy, silver, and silver alloy) on a high molecular material substrate (such as a substrate of polypropylene (PP), polyethylene terephthalate (PET), polybutylene terephthalate (PBT), polystyrene (PS), or polyethylene (PE)).

In the present application, the positive electrode material is a compound into/from which Na⁺ can be reversibly intercalated/deintercalated. As an example, the positive electrode active material includes a transition metal oxide, a polanionic compound, a prussian blue analogue, or the like.

In some embodiments, the positive electrode active material is a transition metal oxide. As an example, a sodium-containing composite oxide represented by NaₓMO₂ or Na_{y}M₂O₄ (wherein M is a transition metal, 0≤x≤1 and 0≤y≤2), a spinel oxide, a metal chalcogenide of a layered structure, an olivine structure, or the like may be enumerated. For example, a sodium-cobalt oxide such as NaCoO₂, a sodium-manganese oxide such as NaMn₂O₄, a sodium-nickel oxide such as NaNiO₂, a sodium-titanium oxide such as Na_{4/3}Ti_{5/3}O₄, a sodium-manganese-nickel composite oxide, a sodium-manganese-nickel-cobalt composite oxide; and a material having an olivine crystal structure such as NaMPO₄ (M=Fe, Mn, or Ni) may be enumerated.

In some embodiments, optionally, the positive electrode active material is a sodium-containing composite oxide of a layered structure or a spinel structure, for example, a sodium-manganese-nickel composite oxide represented by NaCoO₂, NaMn₂O₄, NaNiO₂, or NaNi_{1/2}Mn_{1/2}O₂, a sodium-manganese-nickel-cobalt composite oxide represented by NaNi_{1/3}Mn_{1/3}Co_{1/3}O₂ or NaNi_{0.6}Mn_{0.2}Co_{0.2}O₂, or a sodium-containing composite oxide such as NaNi_{1-x-y-z}CoₓAl_{y}Mg_{z}O₂. Further, a sodium-containing composite oxide obtained by replacing some constituent elements of the above sodium-containing composite oxide with an additional element such as Ge, Ti, Zr, Mg, Al, Mo, or Sn is also included in the scope of the present application.

In some embodiments, optionally, the positive electrode active material is a polanionic compound. As an example, the polyanionic compound may be a class of compounds having sodium ions, transition metal ions, and a tetrahedral (YO₄)ⁿ⁻ anionic unit. The transition metal may be at least one of Mn, Fe, Ni, Co, Cr, Cu, Ti, Zn, V, Zr, and Ce; Y may be at least one of P, S, and Si; and n represents a valence state of (YO₄)ⁿ⁻ . The polyanionic compound may further be a class of compounds having sodium ions, transition metal ions, a tetrahedral (YO₄)ⁿ⁻ anionic unit, and a halide anion. The transition metal may be at least one of Mn, Fe, Ni, Co, Cr, Cu, Ti, Zn, V, Zr, and Ce; Y may be at least one of P, S, and Si; n represents a valence state of (YO₄)ⁿ⁻; and the halogen may be at least one of F, Cl, or Br. The polyanionic compound may further be a class of compounds having sodium ions, a tetrahedral (YO₄)ⁿ⁻ anionic unit, a polyhedral unit (ZO_{y})^{m+}, and optionally a halide anion. Y may be at least one of P, S, and Si, n represents a valence state of (YO₄)ⁿ⁻; Z represents a transition metal, and may be at least one of Mn, Fe, Ni, Co, Cr, Cu, Ti, Zn, V, Zr, and Ce, m represents a valence state of (ZO_{y})^{m+}; and the halogen may be at least one of F, Cl, and Br. The polyanionic compound is, for example, at least one of NaFePO₄, Na₃V₂(PO₄)₃, NaM'PO₄F (M' is one or more of V, Fe, Mn, and Ni), and Na₃(VO_{y})₂(PO₄)₂F_{3-2y} (0≤y≤1).

In some embodiments, optionally, the positive electrode active material is a prussian blue analogue. As an example, the prussian blue compound may be a class of compounds having sodium ions, transition metal ions, and cyanide ions (CN). The transition metal may be at least one of Mn, Fe, Ni, Co, Cr, Cu, Ti, Zn, V, Zr, and Ce. The prussian blue compound is, for example, at least one of NaₐMe_{b}Me'_{c}(CN)₆, wherein Me and Me' are each independently at least one of Ni, Cu, Fe, Mn, Co, and Zn, 0<a≤2, 0<b<1, and 0<c<1.

In some embodiments, the positive electrode film layer further optionally comprises a binder. As an example, the binder may comprise at least one of polyvinylidene fluoride (PVDF), polytetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, and fluoroacrylate resin.

In some embodiments, optionally, the binder is 0.1-3.5%, optionally 0.5-2.5%, of a total weight of the positive electrode film layer.

In some embodiments, the positive electrode film layer further optionally comprises a conductive agent. As an example, the conductive agent may comprise at least one of superconducting carbon, acetylene black, carbon black, Ketjen black, carbon dot, carbon nanotube, graphene, and carbon nanofiber.

In some embodiments, optionally, the conductive agent is 0.05-5%, optionally 0.5-3%, of the total weight of the positive electrode film layer.

In some embodiments, the positive electrode plate may be prepared by: dispersing the above ingredients, such as the positive electrode active material, the conductive agent, the binder, and any other ingredient, for preparing the positive electrode plate in a solvent (such as N-methyl pyrrolidone) to form a positive electrode slurry; and coating the positive electrode slurry on the positive electrode current collector, drying, and cold pressing, to obtain the positive electrode plate.

### [Negative electrode plate]

The negative electrode plate comprises a negative electrode current collector and a negative electrode film layer arranged on at least one surface of the negative electrode current collector, and the negative electrode film layer comprises a negative electrode active material.

As an example, the negative electrode current collector has two surfaces opposite in its own thickness direction, and the negative electrode film layer is arranged on either one or both of the two opposite surfaces of the negative electrode current collector.

In some embodiments, the negative electrode current collector can be a metal foil or a composite current collector. For example, a copper foil can be used as the metal foil. The composite current collector may comprise a high molecular material substrate and a metal layer formed on at least one surface of the high molecular material substrate. The composite current collector may be formed by forming a metal material (such as copper, copper alloy, nickel, nickel alloy, titanium, titanium alloy, silver, and silver alloy) on the high molecular material substrate (such as a substrate of polypropylene (PP), polyethylene terephthalate (PET), polybutylene terephthalate (PBT), polystyrene (PS), or polyethylene (PE)).

In some embodiments, the negative electrode may include a sodium metal negative electrode (including negative electrode free), a carbon material negative electrode, and other non-carbon material negative electrodes.

It should be noted that in the present application, the "negative electrode free" means that a secondary battery does not need to use a negative electrode material, thereby showing a higher energy density (based on same energy). Without being confined to any theory, the working principle of the negative electrode free battery is as follows: in a charging process, sodium ions in a sodium-rich positive electrode material pass through a separator, and combine with electrons transmitted through an external circuit to form metal sodium, which deposits on a current collector; and on the contrary, in a discharging process, the metal sodium on the current collector is dissolved, returns to the electrolyte solution, passes through the separator, and is then re-intercalated into the positive electrode material.

In some embodiments, the negative electrode active material may include at least one of: artificial graphite, natural graphite, soft carbon, hard carbon, a silicon-based material, a tin-based material, lithium titanate, and the like. The silicon-based material may be selected from at least one of elemental silicon, silicon-oxygen compound, silicon-carbon composite, silicon-nitrogen composite, and silicon alloy. The tin-based material may be selected from at least one of elemental tin, tin-oxygen compound, and tin alloy. However, the present application is not limited to these materials, and other conventional materials useful as negative electrode active materials for batteries can also be used. It is possible to use only one of these negative electrode active materials alone, or to use more than two in combination.

In some embodiments, the negative electrode active material comprises hard carbon.

In some embodiments, the negative electrode film layer further optionally comprises a binder. The binder may be selected from at least one of styrene butadiene rubber (SBR), polyacrylic acid (PAA), sodium polyacrylate (PAAS), polyacrylamide (PAM), polyvinyl alcohol (PVA), sodium alginate (SA), polymethacrylic acid (PMAA), and carboxymethyl chitosan (CMCS).

In some embodiments, optionally, the binder is 0.1-3.5%, optionally 0.5-2.5%, of a total weight of the negative electrode film layer.

In some embodiments, the negative electrode film layer further optionally comprises a conductive agent. The conductive agent may be selected from at least one of superconducting carbon, acetylene black, carbon black, Ketjen black, carbon dot, carbon nanotube, graphene, and carbon nanofiber.

In some embodiments, optionally, the conductive agent is 0.05-5%, optionally 0.5-3%, of the total weight of the negative electrode film layer.

In some embodiments, the negative electrode plate may be prepared by: dispersing the above ingredients, such as the negative electrode active material, the conductive agent, the binder, and any other ingredient, for preparing the negative electrode plate in a solvent (such as deionized water) to form a negative electrode slurry; and coating the negative electrode slurry on the negative electrode current collector, drying, and cold pressing, to obtain the negative electrode plate.

### [Separator]

In some embodiments, the secondary battery further comprises a separator. The type of the separator is not particularly limited in the present application, and any well-known separator with a porous structure having good chemical stability and mechanical stability may be selected.

In some embodiments, the material of the separator may be selected from at least one of glass fiber, non-woven cloth, polyethylene, polypropylene, and polyvinylidene fluoride. The separator may be a single-layer film or a multi-layer composite film, and is not particularly limited. When the separator is a multilayer composite film, the material in each layer may be identical or different, which is not particularly limited.

In some embodiments, the positive electrode plate, the negative electrode plate, and the separator can be made into an electrode assembly by a winding process or a stacking process.

In some embodiments, the secondary battery may comprise an outer package. The outer package can be used to encapsulate the above electrode assembly and an electrolyte.

In some embodiments, the outer package of the secondary battery may be a hard case, such as a hard plastic case, an aluminum case, a steel case, and the like. The outer package of the secondary battery may also be a soft pack, such as a bag-type soft pack. The material of the soft pack may be a plastic, and examples of the plastic include polypropylene, polybutylene terephthalate polybutylene succinate, and the like.

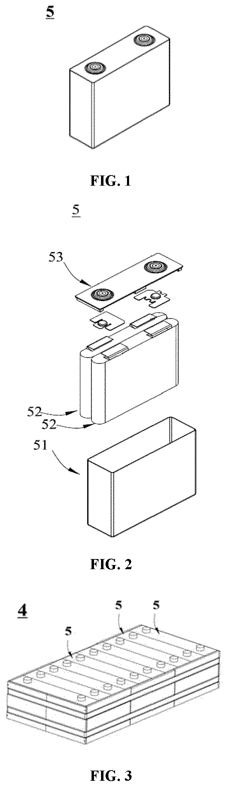

The shape of the secondary battery is not particularly limited in the present application, and may be a cylinder, a square, or any other shape. For example, Fig. 1 shows a secondary battery 5 with a square structure as an example.

In some embodiments, referring to Fig. 2, the outer package may comprise a case 51 and a cover plate 53. The case 51 may comprise a bottom plate and a side plate connected to the bottom plate, which enclose to form an accommodating cavity. The case 51 has an opening that communicates with the accommodating cavity, and the cover plate 53 can cover the opening to close the accommodating cavity. The positive electrode plate, the negative electrode plate, and the separator may form an electrode assembly 52 by a winding process or a stacking process. The electrode assembly 52 is encapsulated within the accommodating cavity. The electrolyte solution impregnates the electrode assembly 52. The number of electrode assemblies 52 comprised in the secondary battery 5 may be one or more, and may be selected by those skilled in the art according to specific actual requirements.

In some embodiments, the secondary batteries may be assembled into a battery module, the number of the secondary batteries included in the battery module may be one or more, and the specific number may be selected by those skilled in the art according to the application and capacity of the battery module.

Fig. 3 shows a battery module 4 as an example. Referring to Fig. 3, in the battery module 4, a plurality of secondary batteries 5 can be sequentially arranged along a length direction of the battery module 4. Of course, any other arrangements are also possible. The plurality of secondary batteries 5 may further be fixed by fasteners.

Optionally, the battery module 4 may further include a shell having an accommodating space, in which the plurality of secondary batteries 5 is accommodated.

In some embodiments, the battery modules may be further assembled into a battery pack, the number of battery modules comprised in the battery pack may be one or more, and the specific number may be selected by those skilled in the art based on the application and capacity of the battery pack.

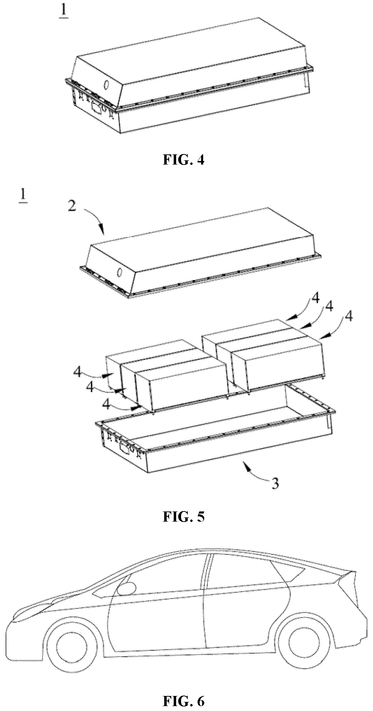

Fig. 4 and Fig. 5 are a battery pack 1 as an example. Referring to Fig. 4 and Fig. 5, the battery pack 1 may comprise a battery box and a plurality of battery modules 4 provided in the battery box. The battery box comprises an upper box 2 and a lower box 3, wherein the upper box 2 can cover the lower box 3 and forms an enclosed space for accommodating the battery module 4. The plurality of battery modules 4 may be arranged in the battery box in any manner.

In addition, the present application further provides an electrical apparatus, comprising at least one of the secondary battery, the battery module, or the battery pack provided in the present application. The secondary battery, the battery module, or the battery pack can be used as a power source for the electrical apparatus, and can also be used as an energy storage unit for the electrical apparatus. The electrical apparatus may include, but is not limited to, a mobile device (such as a mobile phone, and a laptop, etc.), an electric vehicle (such as an all-electric vehicle, a hybrid electric vehicle, a plug-in hybrid electric vehicle, an electric bicycle, an electric scooter, an electric golf cart, and an electric truck, etc.), an electric train, a ship, a satellite, and an energy storage system, etc.

For the electrical apparatus, the secondary battery, the battery module, or the battery pack may be selected according to its use requirements.

Fig. 6 is an electrical apparatus as an example. The electrical apparatus is an all-electric vehicle, a hybrid electric vehicle, a plug-in hybrid electric vehicle, or the like. In order to meet the requirements of the electrical apparatus for high power and high energy density of secondary batteries, a battery pack or a battery module may be used.

As another example, the apparatus may be a mobile phone, a tablet, a laptop, etc. The apparatus is generally required to be light and thin, and may use a secondary battery as a power source.

### Examples

Examples of the present application will be described below. The examples described below are illustrative, are merely used to explain the present application, and should not be construed as limitation on the present application. Where no specific techniques or conditions are specified in the examples, the techniques or conditions described in literatures of the art or the product specifications are prevailing. Where manufacturers are not specified, the reagents or instruments used are conventional products and are commercially available.

### Example 1

### Preparation of an electrolyte solution

In an argon atmosphere within a glove box (H₂O<0.1 ppm, O₂<0.1 ppm), sodium difluoro(oxalato)borate (NaDFOB) and 1,1,2,2-tetrafluoroethyl-2,2,3,3-tetrafluoropropyl ether were dissolved in an organic solvent ethylene glycol dimethyl ether, and the mixture was sufficiently stirred, so that a content of the 1,1,2,2-tetrafluoroethyl-2,2,3,3-tetrluoropropyl ether was 5 wt% (based on a total weight of the electrolyte solution), and a concentration of the sodium difluoro(oxalato)borate was 1.5 mol/L, thus providing the electrolyte solution of Example 1, wherein a content of the ethylene glycol dimethyl ether was 83.5 wt% based on the total weight of the electrolyte solution.

### Preparation of a sodium-ion secondary battery

### [Preparation of a positive electrode plate]

A binder (10 wt% polyvinylidene fluoride) (based on a total weight of a whole slurry formula system excluding N-methylpyrrolidone, the same below) was fully dissolved in N-methyl pyrrolidone. A conductive agent (10 wt% carbon black) and 80 wt% positive electrode active material Na₄Fe₃(PO₄)₂(P₂O₇) were added to make a sufficiently dispersed slurry. The slurry was uniformly coated on a surface of an aluminum foil, which was then transferred to a vacuum drying oven for complete drying. The resulting electrode plate was rolled, and then die cut to provide the positive electrode plate. In the resulting positive electrode plate, a coating surface density of the positive electrode slurry was 0.3 g/1540.25 mm².

### [Preparation of a negative electrode plate]

Preparation of a carbon material coating: first, 5 g of a hard carbon material was transferred into 50 mL of a mixed solution of concentrated sulfuric acid (mass fraction: 98.3%) and concentrated nitric acid (mass fraction: 68%) at a volume ratio of 3:1, and the mixture was stirred for 4 h. Then, the carbon material was carefully taken out, washed with deionized water, filtered, transferred into a drying oven, and dried at 80°C. 2 g of the resulting carbon material and 3 g of styrene butadiene rubber (SBR) were added into N-methyl pyrrolione, and the mixture was stirred to form a uniform slurry. Then, the slurry was coated on a copper foil, and dried to provide the carbon material coating with a coated weight of 0.010 g/1540.25 mm².

Preparation of an active material of sodium metal alloy: in an Ar atmosphere, metal sodium was heated to 200°C in a stainless steel crucible for complete melting. Then, powder of a sodium-bismuth alloy component was added into liquid metal sodium, and the mixture was fully stirred for 2 h to ensure that the metal powder was uniformly mixed with the liquid metal sodium. The mixture was cooled in an Ar protective atmosphere to provide the active material of sodium metal alloy. A content of bismuth in the active material of sodium metal alloy was 5 wt%.

The active material of sodium metal alloy was composited on the surface of the carbon material coating by cold pressing to provide the sodium metal negative electrode plate. In the resulting negative electrode plate, a surface density of the active material of sodium metal alloy on the carbon material coating was 0.025 g/1540.25 mm².

### [Separator]

A polypropylene film (purchased from Shanghai SEMCORP) was used as the separator.

The above positive electrode plate, the above separator, and the above negative electrode plate were stacked in order, such that the separator was between the positive electrode and the negative electrode to serve for separation, and the above electrolyte solution was added to assemble a trilayer stacked battery. A size of the resulting secondary battery was 60 mm×55 mm×5 mm.

### Examples 2-20

Other steps of Examples 2-20 are same as the steps of Example 1 except that the formula of the electrolyte solution is different, details as per Table 1.

### Comparative Examples 1-4

Other steps of Comparative Examples 1-4 are same as the steps of Example 1 except that the formula of the electrolyte solution is different.

### Battery performance test method

### 1. Electrochemical window

The electrolyte solution needs to be stable in an operating voltage range of the battery, and an electrochemically stable voltage range of the electrolyte solution is usually called the electrochemical window.

A cyclic voltammetry curve was mainly tested in the electrochemical window test. A potential of a to-be-tested electrode was controlled for scanning towards a negative direction of potential from Ei (starting potential) at a rate V (1 mV/s), and the scanning direction was changed at time t (t was automatically generated by the test system according to the voltage range) for back-scanning to the starting potential at the same rate. Then, the potential was re-changed, the scanning was repeated for 50 cycles, and a region without occurrence of a redox peak was the electrochemical window obtained in the test.

### 2. coulombic efficiency

Example 1 was taken as an example: the resulting sodium-ion secondary battery was charged at a constant current of 1/3C to 4.15V at 25°C, then charged at a constant voltage of 4.15V until the current was reduced to 0.05C to obtain an initial charge capacity (Cc); and then discharged at a constant current of 1/3C to 2.5V to obtain an initial discharge capacity (Cd). The coulombic efficiency of the sodium-ion battery was computed as per the formula below. coulombic efficiency of sodium-ion battery=initial discharge capacity (Cd)/initial charge capacity (Cc)×100%.

The testing processes in comparative examples and other examples are the same as above.

### 3. Capacity retention rate

Example 1 was taken as an example: the sodium-ion battery was charged at a constant current of 1C to 4.15V at 25°C, then charged at a constant voltage of 4.15V until the current was reduced to 0.05C, and then discharged at a constant current of 1C to 2.5V to obtain a first cycle discharge capacity (Cd1); such charge and discharge were repeated until an n-th cycle to obtain a discharge capacity of the sodium-ion battery after n cycles, which was denoted as Cdn, and the capacity retention rate of the sodium-ion battery was computed as per the formula below. Capacity retention rate=discharge capacity (Cdn) after n cycles/ first cycle discharge capacity (Cd1)×100%.

The testing processes in comparative examples and other examples are the same as above. The data in Table 1 was measured after 200 cycles using the above method.

### 4. Sodium dendrite

The above sodium-ion battery after 200 cycles was disassembled in an argon atmosphere within a glove box (H₂O<0.1 ppm, O₂<0.1 ppm), and the surface appearance of the negative electrode plate was visually observed to determine whether the sodium dendrite was generated. The negative electrode plate with no white spot was ascertained to have no sodium dendrite, the negative electrode plate with sparse white spots was ascertained to have slight sodium dendrite, and the negative electrode plate with dense white spots was ascertained to have serious sodium dendrite.

**Table 1 Experimental Conditions and Test Results in Examples 1-20 and Comparative Examples 1-4**

| No. | Electrolyte salt and concentration (content wt%) | Organic solvent and content (wt%) | Additive and content (wt%) | Mass ratio of electrolyte salt to fluoroalky 1 ether | Electroc hemical window (V) | coulomb ic efficienc y (%) | Capacity retention rate after 200 cycles (%) | Sodiu m dendrit e |
|---|---|---|---|---|---|---|---|---|
| Example 1 | 1.5 M NaDFOB (11.5 wt%) | Ethylene glycol dimethyl ether 83.5 wt% | 1,1,2,2-tetrafluoroethyl-2,2,3,3-tetrafluoropropy l ether 5% | 2.3 | 4.2 | 97.7 | 91.2 | N.A. |
| Example 2 | 1.5 M NaDFOB (11.5 wt%) | Ethylene glycol dimethyl ether 83.5 wt% | Bis(2,2,2-trifluoroethyl) ether 5% | 2.3 | 4.1 | 95.2 | 88.1 | N.A. |
| Example 3 | 1.5 M NaDFOB (11.5 wt%) | Ethylene glycol dimethyl ether 83.5 wt% | 1,1,2,2-tetrafluoroethyl-2,2,2-trifluoroethyl ether 5% | 2.3 | 4.1 | 95.0 | 87.7 | N.A. |
| Example 4 | 1.5 M NaDFOB (11.5 wt%) | Ethylene glycol dimethyl ether 83.5 wt% | Methoxynonaflu orobutane 5% | 2.3 | 3.9 | 93.3 | 86.2 | N.A. |
| Example 5 | 1.5 M NaDFOB (11.5 wt%) | Ethylene glycol dimethyl ether 83.5 wt% | Ethoxynonafluor obutane 5% | 2.3 | 3.9 | 92.9 | 85.6 | N.A. |
| Example 6 | 1.5 M NaDFOB (11.6 wt%) | Ethylene glycol dimethyl ether 86.4 wt% | 1,1,2,2-tetrafluoroethyl-2,2,3,3-tetrafluoropropy l ether 2% | 5.8 | 4.2 | 95.8 | 89.3 | N.A. |
| Example 7 | 1.5 M NaDFOB (12 wt%) | Ethylene glycol dimethyl ether 80 wt% | 1,1,2,2-tetrafluoroethyl-2,2,3,3-tetrafluoropropy l ether 8% | 1.5 | 4.2 | 96.4 | 89.9 | N.A. |
| Example 8 | 1.5 M NaDFOB (11.7 wt%) | Ethylene glycol dimethyl ether 87.3 wt% | 1,1,2,2-tetrafluoroethyl-2,2,3,3-tetrafluoropropy l ether 1% | 11.7 | 4.2 | 92.0 | 84.6 | N.A. |
| Example 9 | 1.5 M NaDFOB (12 wt%) | Ethylene glycol dimethyl ether 68 wt% | 1,1,2,2-tetrafluoroethyl-2,2,3,3-tetrafluoropropy l ether 20% | 0.6 | 4.2 | 92.5 | 85.3 | N.A. |
| Example 10 | 1M NaDFOB (10 wt%) | Ethylene glycol dimethyl ether 85 wt% | 1,1,2,2-tetrafluoroethyl-2,2,3,3-tetrafluoropropy l ether 5% | 2 | 4.2 | 93.9 | 87.5 | N.A. |
| Example 11 | 4 M NaDFOB (20 wt%) | Ethylene glycol dimethyl ether 75 wt% | 1,1,2,2-tetrafluoroethyl-2,2,3,3-tetrafluoropropy l ether 5% | 4 | 4.2 | 93.1 | 86.9 | N.A. |
| Example 12 | 0.5 M NaDFOB (5 wt%) | Ethylene glycol dimethyl ether 90 wt% | 1,1,2,2-tetrafluoroethyl-2,2,3,3-tetrafluoropropy l ether 5% | 1 | 4.2 | 91.2 | 84.6 | N.A. |
| Example 13 | 8M NaDFOB (40 wt%) | Ethylene glycol dimethyl ether 55 wt% | 1,1,2,2-tetrafluoroethyl-2,2,3,3-tetrafluoropropy l ether 5% | 8 | 4.2 | 89.4 | 82.1 | N.A. |
| Example 14 | 1.5 M Sodium bis(oxalato)b orate (11.5 wt%) | Ethylene glycol dimethyl ether 83.5 wt% | 1,1,2,2-tetrafluoroethyl- 2,2,3,3-tetrafluoropropy l ether 5% | 2.3 | 4.2 | 94.3 | 88.5 | N.A. |
| Example 15 | 1.5 M NaDFOB (14.5 wt%) | 1,1,2,2-tetrafluoroethy 1-2,2,3,3-tetrafluoroprop yl ether 80.5% | Ethylene glycol diethyl ether 5% | 0.18 | 4.2 | 91.4 | 84.9 | N.A. |
| Example 16 | 1.5 M NaDFOB (12.4 wt%) | Bis(2,2,2-trifluoroethyl) ether 47.6 wt% | Methyltetrahydr ofuran 40% | 0.26 | 4.2 | 89.6 | 82.5 | N.A. |
| Example 17 | 1.5 M Sodium tetrakis(triflu oromethyl)bo rate (23 wt%) | Ethylene glycol dimethyl ether 72 wt% | 1,1,2,2-tetrafluoroethyl-2,2,3,3-tetrafluoropropy l ether 5% | 4.6 | 4.2 | 92.8 | 86.5 | N.A. |
| Example 18 | 1.5 M Sodium dicyano(oxal ato)borate (12.5 wt%) | Ethylene glycol dimethyl ether 82.5 wt% | 1,1,2,2-tetrafluoroethyl-2,2,3,3-tetrafluoropropy l ether 5% | 2.5 | 4.2 | 91.0 | 84.3 | N.A. |
| Example 19 | 1.5 M NaDFOB (11.5 wt%) | Ethylene glycol dimethyl ether 71.6 wt%, dimethyl carbonate 11.9 wt% | 1,1,2,2-tetrafluoroethyl-2,2,3,3-tetrafluoropropy l ether 5% | 2.3 | 4.2 | 86.3 | 76.4 | N.A. |
| Example 20 | 1.5 M NaDFOB (11.5 wt%) | Ethylene glycol dimethyl ether 41.75 wt%, diethyl carbonate 41.75 wt% | 1,1,2,2-tetrafluoroethyl-2,2,3,3-tetrafluoropropy l ether 5% | 2.3 | 4.2 | 83.4 | 74.6 | Slight |
| Compar ative Example 1 | 1.5 M NaPF6 (25.2 wt%) | Ethylene glycol dimethyl ether 69.8 wt% | 1,1,2,2-tetrafluoroethyl-2,2,3,3-tetrafluoropropy l ether 5% | / | 3.7 | 82.3 | 71.2 | Slight |
| Compar ative Example 2 | 1.5 M NaDFOB (24 wt%) | Ethylene glycol dimethyl ether 76 wt% | / | / | 3.7 | 84.5 | 73.6 | Slight |
| Compar ative Example 3 | 1.5 M NaPF6 (25.2 wt%) | Ethylene glycol dimethyl ether 74.8 wt% | / | / | 3.6 | 81.4 | 70.4 | Seriou s |
| Compar ative Example 4 | 1.5 M NaDFOB (24 wt%) | Ethylene glycol dimethyl ether 71 wt% | Fluoroethylene carbonate 5% | / | 3.7 | 85.1 | 73.9 | Slight |

As can be seen from Table 1, the sodium-ion secondary battery corresponding to the electrolyte solution comprising the sodium ion of formula NaBOₐFₓR_{y}^{-z}, the fluoroalkyl ether, and other ethers except for the fluoroalkyl ether has a broader electrochemical window, coulombic efficiency, cycling performance, and safety performance. The concentration of the sodium salt of formula NaBOₐFₓR_{y}^{-z}, the content of the fluoroalkyl ether, and its type can be further regulated to further improve the performance of the secondary battery. Further, as can be seen from Table 1, the fluoroalkyl ether is more conducive to improving the battery performance than carbonate additives.

It should be noted that the present application is not limited to the above embodiments. The above embodiments are merely illustrative, and embodiments having substantively the same composition and having the same effects as the technical ideas within the scope of the technical solutions of the present application are all included in the technical scope of the present application. In addition, without departing from the scope of the subject matter of the present application, various modifications that can be conceived by those skilled in the art are applied to the embodiments, and other modes constructed by combining some of the constituent elements of the embodiments are also included in the scope of the present application.

## Claims

1. An electrolyte solution for a sodium-ion battery, comprising
a sodium salt of formula NaBOₐFₓR_{y}^{-z}, wherein 2a+x+y×z=2 or 4, 0≤a≤3, 0≤x≤4, 0≤y, 0≤z, 0≤y×z≤4, and R is selected from one or more of oxalato 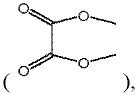 phenyl, cyano, C₁-C₆-fluoroalkyl such as trifluoromethyl, pentafluoroethyl, or 2,2,2-trifluoroethyl, or C₁-C₆-alkoxy such as methoxy or ethoxy;
a fluoroalkyl ether; and
other ethers except for the fluoroalkyl ether, wherein optionally, the other ethers are selected from one or more of an aliphatic ether with 4-20 carbon atoms, an alicyclic ether with 3-8 carbon atoms, an aromatic ether with 7-20 carbon atoms, or a crown ether.

2. The electrolyte solution for a sodium-ion battery according to claim 1, wherein the electrolyte solution for a sodium-ion battery satisfies one or more conditions of:
(1) a content of the sodium salt of formula NaBOₐFₓR_{y}^{-z} is 5-40 wt%, optionally 10-20 wt%;
(2) a content of the fluoroalkyl ether is 1-82 wt%, optionally 1-48 wt%, more optionally 1-20 wt%, and most optionally 2-8 wt%;
(3) a sum of the content of the sodium salt of formula NaBOₐFₓR_{y}^{-z} and the content of the fluoroalkyl ether is 10-95 wt%, optionally 10-60 wt%, and more optionally 12-28 wt%;
(4) a content of the other ethers is 5-90 wt%, optionally 40-90 wt%, and more optionally 72-88 wt%; and
(5) a concentration of the sodium salt of formula NaBOₐFₓR_{y}^{-z} is 0.5-8 mol/L, optionally 1-4 mol/L;
wherein the contents in (1)-(4) are all based on a total weight of the electrolyte solution for a sodium-ion battery, and a total weight of the components in the electrolyte solution for a sodium-ion battery is 100 wt%.

3. The electrolyte solution for a sodium-ion battery according to claim 1 or 2, wherein
a mass ratio of the sodium salt of formula NaBOₐFₓR_{y}^{-z} to the fluoroalkyl ether is 1:0.08-6, optionally 1:0.08-4, and more optionally 1:0.1-0.8; and
optionally, a mass ratio of the fluoroalkyl ether to the other ethers except for the fluoroalkyl ether is 1:0.06-90, optionally 1:2-90, and more optionally 1:9-44.

4. The electrolyte solution for a sodium-ion battery according to any one of claims 1 to 3, wherein the sodium salt of formula NaBOₐFₓR_{y}^{-z} is selected from one or more of sodium difluoroborate, sodium tetrafluoroborate, sodium bis(oxalato)borate, sodium difluoro(oxalato)borate, sodium tetraphenylborate, sodium tetracyanoborate, sodium tetrakis(trifluoromethyl)borate, sodium bis(trifluoromethyl)difluoroborate, sodium pentafluroroethyltrifluoroborate, sodium dicyano(oxalato)borate, sodium methoxytricyanoborate, sodium ethoxytricyanoborate, sodium tetramethooxyborate, sodium tetraethoxyborate, or sodium cyanotris(2,2,2-trifluoroethyl)borate; and
is optionally one or more of sodium bis(oxalato)borate, sodium difluoro(oxalato)borate, sodium tetrakis(trifluoromethyl)borate, sodium bis(trifluoromethyl)difluoroborate, or sodium dicyano(oxalato)borate.

5. The electrolyte solution for a sodium-ion battery according to any one of claims 1 to 4, wherein
the fluoroalkyl ether is selected from one or more of 1,1,2,2-tetrafluoroethyl-2,2,3,3-tetrafluoropropyl ether, bis(2,2,2-trifluoroethyl) ether, 1, 1,2,2-tetrafluoroethyl-2,2,2-trifluoroethyl ether, methoxynonafluorobutane, or ethoxynonafluorobutane.

6. The electrolyte solution for a sodium-ion battery according to any one of claims 1 to 5, wherein the other ethers are selected from one or more of ethylene glycol diethyl ether, ethylene glycol dimethyl ether, diethylene glycol dimethyl ether, tridiethylene glycol dimethyl ether, tetraethylene glycol dimethyl ether, 1,3-dioxolane, tetrahydrofuran, methyltetrahydrofuran, diphenyl ether, or crown ether; and
are optionally one or more of ethylene glycol diethyl ether, ethylene glycol dimethyl ether, or diethylene glycol dimethyl ether.

7. The electrolyte solution for a sodium-ion battery according to any one of claims 1 to 6, wherein the electrolyte solution for a sodium-ion battery comprises no carbonate; or
if a carbonate is present, a ratio of a percentage mass content of the carbonate to a percentage mass content of all ether solvents is from 1:9 to 3:2, optionally from 1:6 to 1:1.

8. A sodium-ion secondary battery, comprising the electrode solution according to any one of claims 1 to 7.

9. A battery module, comprising the sodium-ion secondary battery according to claim 8.

10. A battery pack, comprising at least one of the sodium-ion secondary battery according to claim 8 or the battery module according to claim 9.

11. An electrical apparatus, comprising at least one of the sodium-ion secondary battery according to claim 8, the battery module according to claim 9, or the battery pack according to claim 10.
